# EUROPEAN PATENT APPLICATION

(11) **EP 4 643 924 A1**
(43) Date of publication of application: **05.11.2025**
(21) Application number: 23910957.2
(22) Date of filing: 29.12.2023
(51) Int. Cl.: A61M 60/178, H02K 5/16, H02K 5/10, H02K 5/124, A61M 60/216, A61M 60/411, A61M 60/855, A61M 60/871

(54) **MOTOR ASSEMBLY OF ACTIVE INTERVENTIONAL MEDICAL INSTRUMENT**

(30) Priority: 30.12.2022 CN 202211733043
(71) Applicant: Forqaly Medical (Shanghai) Co., Ltd., Shanghai 201318 (CN)
(72) Inventor: LI, Hansong, Shanghai 201318 (CN); TANG, Zhirong, Shanghai 201318 (CN)
(74) Representative: Valea AB
(86) International application number: PCT/CN2023/143389
(87) International publication number: WO 2024/141044

(57) **Abstract**

The present application discloses a motor assembly of an active interventional medical instrument. The motor assembly comprises a motor main body, a perfusion tube, and a return tube. The motor main body comprises a stator assembly, a rotor assembly, and a housing. The stator assembly and the rotor assembly are arranged within the housing. The rotor assembly is sleeved with the stator assembly. The gap between the rotor assembly and the stator assembly forms a first cavity. The rotor assembly comprises a rotating shaft and a bearing. The rotating shaft extends in a first direction, and the rotating shaft is sleeved with the bearing and rotates, by means of the bearing, relative to the housing.

## Description

### CROSS-REFERENCE TO RELATED APPLICATIONS

This application claims the priority to Chinese Patent Application No. 202211733043.1 filed on December 30, 2022, and entitled "MOTOR ASSEMBLY OF ACTIVE INTERVENTIONAL MEDICAL DEVICE", which is incorporated herein by reference in its entirety.

### TECHNICAL FIELD

The present disclosure belongs to the technical field of medical devices, and in particular, relates to a motor assembly of an active interventional medical device.

### BACKGROUND

During cardiac surgery, due to the patient's disease or the need of surgery, the patient's heart function becomes weak and the blood-pumping ability is insufficient. In this case, it is necessary to insert an active interventional medical device, such as a ventricular assist device, into the heart to assist the heart in pumping blood. The existing ventricular assist device utilizes the principle of heart pumping blood to pump out the blood in the heart through a pumping mechanism, and to guide the blood to the aorta out of the heart to flow to the whole body.

The existing ventricular assist device includes a catheter and a blood-pumping mechanism, and the blood-pumping mechanism is disposed at a distal end (the end away from the operator or the doctor) of the catheter. The catheter is inserted into the left ventricle of the patient's heart through the femoral artery, the axillary artery, or the carotid artery, and the blood-pumping mechanism then enters the patient's left atrium. The blood-pumping mechanism can also be inserted into the right ventricle of the patient's heart through a vein such as the femoral vein by means of the catheter.

The blood-pumping mechanism includes a motor assembly of the active interventional medical device, and the motor assembly of the active interventional medical device is provided with a bearing disposed therein. The bearing will generate very tiny particles when the motor assembly of the active interventional medical device rotates at a high speed. The particles will flow into the patient's vessels with the perfusion solution in the perfusion catheter, which will harm the human body.

### SUMMARY

The embodiments of the present disclosure provide a motor assembly of an active interventional medical device, which may reduce a total amount of particles flowing into the patient's vessels.

In a first aspect, some embodiments of the present disclosure provide a motor assembly of an active interventional medical device. The motor assembly of the active interventional medical device includes a motor body, a perfusion catheter, and a reflux catheter. The motor body includes a stator assembly, a rotor assembly, and a housing. The stator assembly and the rotor assembly are disposed in the housing. The stator assembly is fitted on the rotor assembly. A gap between the rotor assembly and the stator assembly forms a first cavity. The rotor assembly includes a rotor shaft extending in a first direction and a bearing fitted on the rotor shaft. The rotor shaft is rotatable relative to the housing through the bearing. At least part of the perfusion catheter is disposed in the housing, and the perfusion catheter is in communication with the first cavity. At least part of the reflux catheter is disposed in the housing. The reflux catheter is isolated from the perfusion catheter and is in communication with the first cavity. External perfusion solution flows through the perfusion catheter, the bearing, and the reflux catheter in sequence in such a manner that the external perfusion solution flows out of the first cavity.

According to any one embodiment in the first aspect provided by the present disclosure, an inner wall of the housing is provided with a flow groove recessed towards the rotor shaft, the flow groove is in communication with the first cavity, and at least part of the flow groove serves as a channel for the perfusion catheter.

According to any one embodiment in the first aspect provided by the present disclosure, the perfusion catheter is disposed at a proximal end of the housing, a gap between the stator assembly and the housing forms a second cavity, and the first cavity is in communication with the perfusion catheter through the second cavity.

According to any one embodiment in the first aspect provided by the present disclosure, the gap between the stator assembly and the housing is divided into two compartments of which one serves as the second cavity and the other one serves as a cavity for accommodating a wiring harness.

According to any one embodiment in the first aspect provided by the present disclosure, the rotor shaft extends out of the housing from a distal end of the housing, and the housing includes a sealing cover located at the distal end of the housing, fitted on the rotor shaft, and sealing the distal end of the housing.

According to any one embodiment in the first aspect provided by the present disclosure, a side of the sealing cover facing towards an interior of the housing is provided with a communication groove communicating the first cavity with the perfusion catheter.

According to any one embodiment in the first aspect provided by the present disclosure, a plurality of the bearings includes a first bearing fitted on the rotor shaft and disposed at a proximal end of the housing, and a second bearing fitted on the rotor shaft and disposed at the distal end of the housing, and the perfusion solution flows through the perfusion catheter, the communication groove, the first bearing, and the reflux catheter in sequence in such a manner that the perfusion solution flows out of the first cavity.

According to any one embodiment in the first aspect provided by the present disclosure, the second bearing is embedded in the sealing cover, and the perfusion solution flows through the perfusion catheter, the communication groove, the second bearing, the first bearing, and the reflux catheter in sequence in such a manner that the perfusion solution flows out of the first cavity.

According to any one embodiment in the first aspect provided by the present disclosure, the second bearing is located at a side of the sealing cover away from the first cavity.

According to any one embodiment in the first aspect provided by the present disclosure, the housing further includes a base plate located at a proximal end of the housing, isolating the stator assembly and the rotor assembly from external environment, and provided with a second through-hole penetrating the base plate along the first direction, and the first cavity is in communication with the reflux catheter through the second through-hole.

According to any one embodiment in the first aspect provided by the present disclosure, the reflux catheter and the rotor shaft are coaxial, and the reflux catheter is disposed on an extension line of the rotor shaft extending towards the proximal end of the housing.

The motor assembly of the active interventional medical device according to the embodiments of the present disclosure includes the motor body, the perfusion catheter, and the reflux catheter. The motor body includes the stator assembly, the rotor assembly, and the housing. The stator assembly and the rotor assembly are fixed in the housing. The gap between the rotor assembly and the stator assembly forms the first cavity. The rotor assembly includes the rotor shaft and the bearing. The rotor shaft is molded by extending in the first direction, and the bearing is fitted on the rotor shaft. At least part of the perfusion catheter is disposed in the housing, and the perfusion catheter is in communication with the first cavity. At least part of the reflux catheter is disposed in the housing. The reflux catheter is in communication with the first cavity. The reflux catheter is isolated from the perfusion catheter, and the external perfusion solution flows through the perfusion catheter, the bearing, and the reflux catheter in sequence in such a manner that the external perfusion solution flows out of the first cavity. In the present disclosure, the perfusion solution is perfused into the first cavity through the perfusion catheter, which reduces the particles generated when the motor body runs. The perfusion solution flowing through the first cavity and the bearing is recycled by the reflux catheter, which effectively prevents the particles generated when the motor body runs, from entering the human body, and reduces the total amount of the perfusion solution flowing into the patient's body. Thus, the total amount of the particles flowing into the patient's body can be reduced, and the product safety can be improved. In addition, the perfusion solution is recycled after flowing through the interior of the motor, which can bring out the heat generated by the blood-pumping motor and reduce the temperature rise of the motor.

### BRIEF DESCRIPTION OF THE DRAWINGS

In order to describe the technical solutions of the embodiments of the present disclosure more clearly, the accompanying drawings for the embodiments of the present disclosure will be briefly described in the following. For a person skilled in the art, other drawings can be obtained according to these drawings without the creative efforts.
Fig. 1 is a first cross-sectional view of a motor assembly of an active interventional medical device after a perfusion catheter is hidden provided by some embodiments of the present disclosure;
Fig. 2 is a partially enlarged cross-sectional view of the motor assembly of the active interventional medical device in Fig. 1 provided by some examples;
Fig. 3 is a schematic diagram of a sealing cover in Fig. 2 provided by some examples;
Fig. 4 is a side view of a core provided by some examples;
Fig. 5 is a second cross-sectional view of a motor assembly of an active interventional medical device provided by some examples;
Fig. 6 is a partially enlarged cross-sectional view of the motor assembly of the active interventional medical device in Fig. 5 provided by some examples;
Fig. 7 is a schematic diagram of a sealing cover in Fig. 6 provided by some examples;
Fig. 8 is a schematic diagram of a sleeve, a base plate, and a flow groove in Fig. 6 provided by some examples;
Fig. 9 is a schematic diagram of a cover body and the flow groove in Fig. 6 provided by some examples;
Fig. 10 is a cross-sectional view of the cover body in Fig. 6 provided by some examples;
Fig. 11 is a third cross-sectional view of a motor assembly of an active interventional medical device provided by some examples; and
Fig. 12 is a cross-sectional view of the motor assembly of the active interventional medical device along A-A in Fig. 11 provided by some examples.

### Reference signs:

10, motor body; 11, stator assembly; 111, core; 112, winding; 12, rotor assembly; 121, rotor shaft; 122, bearing; 1221, first bearing; 1222, second bearing; 123, permanent magnet; 13, housing; 133, sealing cover; 1331, first through-hole; 1332, communication groove; 1333, first sinking hole; 1334, second sinking hole; 1335, first step; 1336, second step; 1337, third step; 1338, fourth step; 134, sleeve; 1341, base plate; 1342, second through-hole; 1343, fourth through-hole; 1344, first wire pass-through hole; 135, cover body; 1351, third through-hole; 1352, second wire pass-through hole; 136, flow groove; 14, first cavity; 15, second cavity; 16, sealing pad;
20, perfusion catheter;
30, reflux catheter;
40, outflow channel; 41, outflow window; 42, impeller; and
x, first direction.

### DETAILED DESCRIPTION

Features and exemplary embodiments of various aspects of the present disclosure will be described in detail below. In order to make the objects, technical solutions and advantages of the present disclosure more clear, the present disclosure will be further described in detail below in combination with the drawings and specific embodiments. It should be understood that, the specific embodiments described herein are only intended to explain the present disclosure, rather than limiting the present disclosure. For those skilled in the art, the present disclosure can be implemented without some of those specific details. The following description of the embodiments only intends to provide a better understanding of the present disclosure through illustrating examples.

It should be noted that, in the present disclosure, relational terms such as first, second, and the like are used herein merely to distinguish one entity or operation from another without necessarily requiring or implying any actual relationship or order between such entities or operations. Moreover, the terms "include", "contain", or any other variation thereof, are intended to cover a non-exclusive inclusion, such that a process, method, article, or device that includes a series of elements not only includes these elements but also includes other elements not explicitly listed or also includes elements inherent to such process, method, article, or device. An element defined by "include..." does not, without more limitations, preclude additional identical elements in the process, method, article or device that includes the element.

Applicant has found that, in the related art, a bearing is provided in the motor assembly of the active interventional medical device, and the bearing will generate very tiny particles when the motor assembly of the active interventional medical device rotates at a high speed. The particles will flow into the patient's vessels with the perfusion solution in the perfusion catheter, which will harm the human body.

In view of the above problem, a motor assembly of an active interventional medical device is proposed by the applicant.

The motor assembly of the active interventional medical device includes a motor body, a perfusion catheter, and a reflux catheter. The motor body includes a stator assembly, a rotor assembly, and a housing. The stator assembly and the rotor assembly are disposed in the housing. The stator assembly is fitted on the rotor assembly. A gap between the rotor assembly and the stator assembly forms a first cavity. The rotor assembly includes a rotor shaft extends in a first direction and a bearing fitted on the rotor shaft. The rotor shaft is rotatable relative to the housing through the bearing. At least part of the perfusion catheter is disposed in the housing, and the perfusion catheter is in communication with the first cavity. At least part of the reflux catheter is disposed in the housing. The reflux catheter is isolated from the perfusion catheter and is in communication with the first cavity. External perfusion solution flows through the perfusion catheter, the bearing, and the reflux catheter in sequence in such a manner that the external perfusion solution flows out of the first cavity.

The motor assembly of the active interventional medical device provided by the present disclosure includes the motor body, the perfusion catheter, and the reflux catheter. The motor body includes the stator assembly, the rotor assembly, and the housing. The stator assembly and the rotor assembly are fixed in the housing. The gap between the rotor assembly and the stator assembly forms the first cavity. The rotor assembly includes the rotor shaft and the bearing. The rotor shaft is molded by extending in the first direction, and the bearing is fitted on the rotor shaft. At least part of the perfusion catheter is disposed in the housing, and the perfusion catheter is in communication with the first cavity. At least part of the reflux catheter is disposed in the housing. The reflux catheter is in communication with the first cavity. The reflux catheter is isolated from the perfusion catheter, and the external perfusion solution flows through the perfusion catheter, the bearing, and the reflux catheter in sequence in such a manner that the external perfusion solution flows out of the first cavity. In the present disclosure, the perfusion solution is perfused into the first cavity through the perfusion catheter, which reduces the particles generated when the motor body runs. The perfusion solution in the first cavity is recycled by the reflux catheter, and at the same time, the bearing is flushed with the perfusion solution, which effectively prevents the particles generated when the motor body runs, from entering the human body, and reduces the total amount of the perfusion solution flowing into the patient's body. Thus, the total amount of the particles flowing into the patient's body can be reduced, and the product safety can be improved. In addition, the perfusion solution flowing through the interior of the motor is recycled, which can bring out the heat generated by the motor body and reduce the temperature rise of the motor body.

The display module provided by the embodiments of the present disclosure will be described hereinafter with reference to the drawings. It should be noted that, an x direction in the drawings is a first direction. In the drawings, for convenience in drawing, the dimensions in the drawings are not necessarily proportional to the actual dimensions.

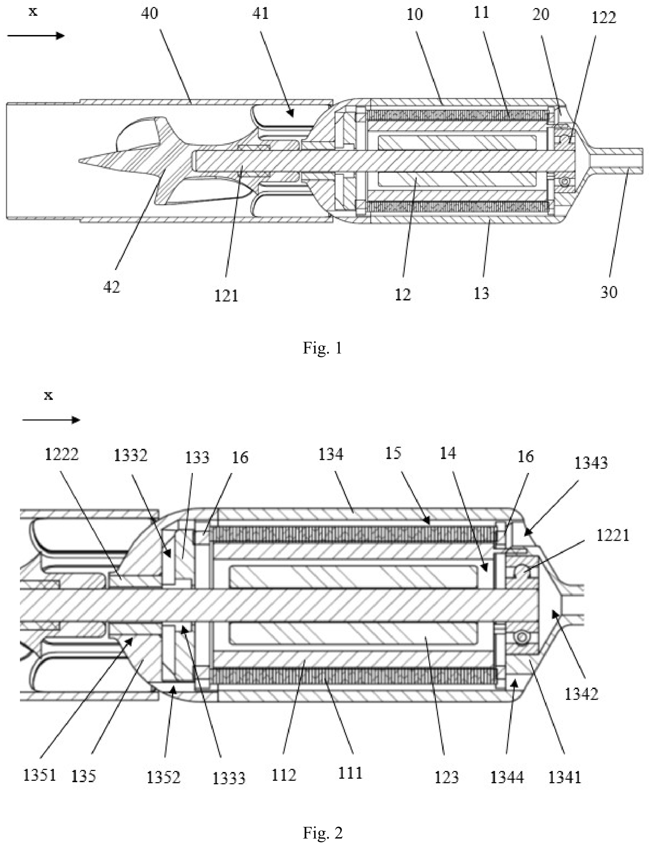

Referring to Figs. 1 and 2, Fig. 1 is a first cross-sectional view of a motor assembly of an active interventional medical device after a perfusion catheter is hidden provided by some embodiments of the present disclosure, and Fig. 2 is a partially enlarged cross-sectional view of the motor assembly of the active interventional medical device in Fig. 1 provided by some examples.

As shown in Figs. 1 and 2, the present disclosure provides a motor assembly of an active interventional medical device. The motor assembly of the active interventional medical device includes a motor body 10, a perfusion catheter 20, and a reflux catheter 30. The motor body 10 includes a stator assembly 11, a rotor assembly 12, and a housing 13. The stator assembly 11 and the rotor assembly 12 are fixed in the housing 13. The stator assembly 11 is fitted on the rotor assembly 12. A gap between the rotor assembly 12 and the stator assembly 11 forms a first cavity 14. The rotor assembly 12 includes a rotor shaft 121 and a bearing 122. The rotor shaft 121 is molded by extending in a first direction (the x direction in the drawings). The rotor shaft 121 is fitted on the bearing 122. The rotor shaft 121 is rotatable relative to the housing 13 by means of the bearing 122. At least part of the perfusion catheter 20 is disposed in the housing 13, and the perfusion catheter 20 is in communication with the first cavity 14. At least part of the reflux catheter 30 is disposed in the housing 13, and the reflux catheter 30 is in communication with the first cavity 14. An external perfusion solution flows through the perfusion catheter 20, the bearing 122, and the reflux catheter 30 in sequence in such a manner that the external perfusion solution flows out of the first cavity 14.

Optionally, the motor assembly of the active interventional medical device has one end connected to an interventional catheter (not shown in the drawings), and the other end connected to an outflow channel 40. One end of the interventional catheter away from the motor body 10 extends out of the patient's body to be connected to other extracorporeal devices, and at least part of the perfusion catheter 20 and at least part of the reflux catheter 30 are accommodated in the interventional catheter. The perfusion solution includes physiological saline and an anticoagulant, and the anticoagulant can be heparin. When the motor assembly of the active interventional medical device is in a blood-pumping state, the perfusion catheter 20 continuously delivers the perfusion solution into the motor body 10, which maintains a certain pressure in the first cavity 14 and prevents blood in the outflow channel 40 from flowing into the first cavity 14. At the same time, the anticoagulant in the perfusion solution reduces the probability of blood coagulation, and thus reduces the probability of failure of the blood-pumping function of the motor body 10 caused by the blood coagulation. The perfusion solution flows into the first cavity 14 through the perfusion catheter 20, and after flushing the bearing 122, flows into the reflux catheter 30 through the gap of the bearing 122, and finally flows out of the patient through the reflux catheter 30, while carrying away particles generated when the bearing 122 rotates at a high speed. The bearing 122 can be a sliding bearing or a ball bearing.

The motor assembly of the active interventional medical device provided by the embodiments includes the motor body 10, the perfusion catheter, and the reflux catheter 30. The motor body 10 includes the stator assembly 11, the rotor assembly 12, and the housing 13, and the stator assembly 11 and the rotor assembly 12 are fixed in the housing 13. The gap between the rotor assembly 12 and the stator assembly 11 forms the first cavity 14. The rotor assembly 12 includes the rotor shaft 121 and the bearing 122. The rotor shaft 121 is molded by extending in the first direction x. The bearing 122 is fitted on the rotor shaft 121. At least part of the perfusion catheter 20 is disposed in the housing 13, and the perfusion catheter 20 is in communication with the first cavity 14. At least part of the reflux catheter 30 is disposed in the housing 13, and the reflux catheter 30 is in communication with the first cavity 14. The reflux catheter 30 is isolated from the perfusion catheter 20. The external perfusion solution flows through the perfusion catheter 20, the bearing 122, and the reflux catheter 30 in sequence in such a manner that the external perfusion solution flows out of the first cavity 14. In the present disclosure, the perfusion solution is perfused into the first cavity 14 through the perfusion catheter 20, which reduces the particles generated when the motor body 10 runs. The perfusion solution in the first cavity 14 is recycled by the reflux catheter 30, and at the same time, the bearing 122 is flushed with the perfusion solution, which carries particles generated in the motor body 10 out of the human body, reduces the harm to the human body, and improves the product safety. In addition, the perfusion solution is recycled after flowing through the interior of the motor body 10, which can bring out the heat generated by the blood-pumping motor and reduce the temperature rise of the motor body 10.

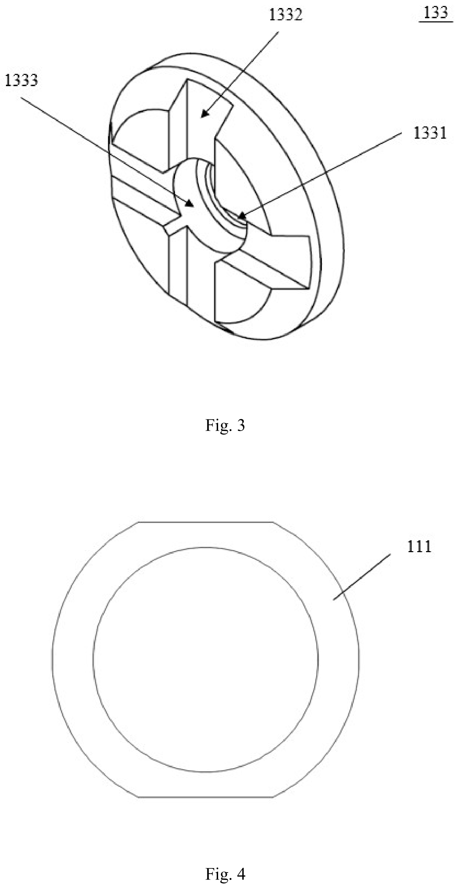

Referring to Figs. 1 to 3, Fig. 3 is a schematic diagram of a sealing cover in Fig. 2 provided by some examples.

As shown in Figs. 1 to 3, in some optional embodiments, the perfusion catheter 20 is disposed at a proximal end of the housing 13. A gap between the stator assembly 11 and the housing 13 forms a second cavity 15, and the first cavity 14 is in communication with the perfusion catheter 20 through the second cavity 15.

Optionally, the proximal end of the housing 13 refers to one end of the housing 13 facing towards an operator or a doctor, and a distal end of the housing 13 refers to the other end of the housing 13 away from the operator or the doctor. The proximal end of the housing 13 is connected to the interventional catheter, and the distal end of the housing 13 faces towards the outflow channel 40. The stator assembly 11 includes a core 111 and a winding 112. The core 111 can wrap around the winding 112, or the winding 112 can wrap around the core 111. When the core 111 wraps around the winding 112, a gap between the core 111 and the housing 13 forms the second cavity 15. When the winding 112 wraps around the core 111, a gap between the winding 112 and the housing 13 forms the second cavity 15. The rotor assembly 12 can include a permanent magnet 123 fitted on the rotor shaft 121, and the permanent magnet 123 is located in the first cavity 14 and rotates synchronously with the rotor shaft 121. When the winding 112 is energized, the winding 112 generates a magnetic field, and the magnetic field interacts with a magnetic field of the permanent magnet 123 so that the permanent magnet 123 and the rotor shaft 121 rotate synchronously, and then the rotor shaft 121 drives the impeller 42 to rotate, thereby realizing the blood-pumping function of the motor assembly of the active interventional medical device.

Referring to Figs. 1 to 4, Fig. 4 is a schematic side view of a core provided by some examples.

As shown in Figs. 1 to 4, in some optional embodiments, the gap between the stator assembly 11 and the housing 13 is divided into two compartments of which one serves as the second cavity 15 and the other one serves as a cavity for accommodating a wiring harness.

Optionally, the core 111 has a cylinder-like shape, and the core 111 is cut axially symmetrically, such that two compartments of a gap are formed between the core 111 and the housing 13, and serve as the second cavity 15 and the cavity for accommodating the wiring harness, respectively. A wiring harness of a pressure sensor (not shown in the drawings) passes through the motor body 10 through the cavity for accommodating the wiring harness and is ultimately connected to an exterior of the patient through the interventional catheter. The pressure sensor is configured to monitor a blood pressure at a position of the ventricular assist device in the patient's body.

In the motor assembly of the active interventional medical device provided by the embodiments, the gap between the stator assembly 11 and the housing 13 is divided into two compartments of which one serves as the cavity for accommodating the wiring harness, so that the wiring harness of the pressure sensor may pass through the interior of the motor body 10, which significantly reduces an outer diameter of the motor assembly of the active interventional medical device compared with a solution where the wiring harness is disposed on an outer edge of the housing 13. In this way, the difficulty for inserting the motor assembly of the active interventional medical device into the patient's body is reduced.

In some optional embodiments, the rotor shaft 121 extends out of the housing 13 from the distal end of the housing 13. The housing 13 includes a sealing cover 133 located at the distal end of the housing 13, fitted on the rotor shaft 121, and sealing the distal end of the housing 13.

Optionally, the outflow channel 40 is provided with an outflow window, and an impeller 42 is provided in the outflow channel 40. The rotor shaft 121 extends out of the housing 13 from the distal end of the housing 13 and then is connected to the impeller 42. When the rotor shaft 121 rotates, the rotor shaft 121 drives the impeller 42 to rotate, and the impeller 42 drives the blood in the outflow channel 40 to flow out from the outflow window 41 to realize the blood-pumping function of the motor assembly of the active interventional medical device. A sealing pad 16 is provided between the sealing cover 133 and the stator assembly 11, and is configured to improve a sealing performance of the first cavity 14.

Optionally, the sealing cover 133 is provided with a first through-hole 1331 penetrating the sealing cover 133 along the first direction x, and the rotor shaft 121 passes through the first through-hole 1331 and then extends out of the housing 13.

In some optional embodiments, the housing 13 includes a sleeve 134 and a cover body 135. The sleeve 134 extends in the first direction x. In the first direction x, the sleeve 134 is provided with an opening (not shown in the drawings) at one end of the sleeve 134 and is provided with a base plate 1341 at the other end. The base plate 1341 is located at the proximal end of the housing, and the base plate 1341 isolates the stator assembly 11 and the rotor assembly 12 from external environment. The base plate 1341 is provided with a second through-hole 1342 and a fourth through-hole 1343 that each penetrate the base plate 1341 along the first direction x, and the first cavity 14 is in communication with the reflux catheter 30 through the second through-hole 1342. The second cavity 15 is in communication with the perfusion catheter 20 through the fourth through-hole 1343, and the perfusion solution flows into the first cavity 14 through the fourth through-hole 1343, the second cavity 15, and a communication groove 1332.

Optionally, the base plate 1341 seals the proximal end of the housing 13, thereby isolating the stator assembly 11 and the rotor assembly 12 from the housing 13. The cover body 135 is connected to the sleeve 134, is configured to close an opening, and is provided with a third through-hole 1351. The sealing cover 133 is mounted on a side of the cover body 135 facing towards the first cavity 14. The first through-hole 1331 and the third through-hole 1351 are coaxial, and the rotor shaft 121 extends out of the housing 13 after passing through the first through-hole 1331 and the third through-hole 1351 in sequence.

Optionally, the housing 13 is made of a stainless steel material, and a side of the cover body 135 facing towards the outflow channel 40 is provided with multiple circular arc transition curved surfaces configured to guide blood to flow out of the outflow channel 40 through the outflow window 41. The sleeve 134 and the cover body 135 can be connected by a process such as welding. Another sealing pad 16 is provided between the stator assembly 11 and the base plate 1341.

In the motor assembly of the active interventional medical device provided by the embodiments, it is convenient to amount the stator assembly 11 and the rotor assembly 12 into the housing 13 with a design where the sleeve 134 is isolated from the cover body 135.

In some optional embodiments, the reflux catheter 30 is disposed on an extension line of the rotor shaft 121 extending towards the proximal end of the housing 13.

In some optional embodiments, a side of the sealing cover 133 facing towards an interior of the housing 13 is provided with the communication groove 1332, and the communication groove 1332 communicates the first cavity 14 with the perfusion catheter 20.

Optionally, multiple communication grooves 1332 are disposed on the sealing cover 133, the communication grooves 1332 communicate the perfusion catheter 20 with the first through-hole 1331, and the perfusion solution flows into the first cavity 14 from the communication grooves 1332 on the sealing cover 133 and the first through-hole 1331 in the sealing cover 133. At the first through-hole 1331, a gap is formed between the first through-hole 1331 and the rotor shaft 121, and part of the perfusion solution can flow into the first cavity 14 through the gap and other part of the perfusion solution can flow into the outflow channel 40 through the gap. A size of the gap between the rotor shaft 121 and the first through-hole 1331 is set in such a manner that most of the perfusion solution is controlled to flow into the first cavity 14, and a small part of the perfusion solution is used to balance a difference between a pressure at the outflow channel 40 and a pressure at the first through-hole 1331 to reduce the blood in the outflow channel 40 flowing into the motor main body 10.

In the motor assembly of the active interventional medical device provided by the embodiments, the first cavity 14 is sealed by the sealing cover 133. The sealing cover 133 is located at the distal end of the housing 13, and the perfusion solution flows into the first cavity 14 from the communication groove 1332 and the first through-hole 1331 at the distal end and flows out of the first cavity 14 from the second through-hole 1342 at the proximal end, which not only balances the pressure at the first through-hole 1331 to reduce a probability of blood flowing into the motor body 10, but also sufficiently flushes the first cavity 14 to reduce the total amount of particles flowing into the patient's body to improve the product safety.

In some optional embodiments, the bearings 122 include a first bearing 1221 and a second bearing 1222 that are both fitted on the rotor shaft 121, the first bearing is disposed at the proximal end of the housing 13, and the second bearing 1222 is disposed at the distal end of the housing 13. The perfusion solution flows through the perfusion catheter 20, the communication groove 1332, the first bearing 1221, and the reflux catheter 30 in sequence in such a manner that the perfusion solution flows out of the first cavity 14.

Optionally, the first bearing 1221 is installed on a side of the base plate 1341 facing towards the first cavity 14, the first bearing 1221 is aligned with the second through-hole 1342, and the perfusion solution flows out of the first cavity through the gap in the first bearing 1221 and the second through-hole 1342. The first bearing 1221 is preferably an open deep groove ball bearing having inner and outer rings made of high-nitrogen steel, a holder made of polyether ether ketone (PEEK), and balls made of ceramic material (not limited to zirconia ceramic). High-nitrogen steel has the characteristics such as high corrosion resistance, high chemical resistance, and high temperature stability. The ceramic balls have the characteristics such as high temperature stability, good self-lubrication, and high wear resistance. These configurations not only ensure a good performance but also makes the generated particles as few as possible.

In the motor assembly of the active interventional medical device provided by the embodiments, the perfusion solution flows into the first cavity 14 from the communication groove 1332 and the first through-hole 1331 that are located at the distal end of the housing 13, and flows out of the first cavity 14 from the first bearing 1221 at the proximal end of the housing 13, which not only balances the pressure at the first through-hole 1331 to reduce a probability of blood flowing into the motor body 10, but also sufficiently flushes the first cavity 14 and the first bearing 1221 to reduce the total amount of particles flowing into the patient's body to improve the product safety.

In some optional embodiments, the second bearing 1222 is disposed on one side of the sealing cover 133 away from the first cavity 14.

Optionally, the sealing cover 133 is provided with a first sinking hole 1333 along the first direction x, and the first sinking hole 1333 is coaxial with the first through-hole 1331, and a diameter of the first sinking hole 1333 is greater than a diameter of the first through-hole 1331. The first sinking hole 1333 is located at a side of the first through-hole 1331 away from the first cavity 14, and the communication groove 1332 communicates the perfusion catheter 20 with the first sinking hole 1333.

Optionally, the second bearing 1222 is installed in the third through-hole 1351, and the rotor shaft 121 passes through the first through-hole 1331, the first sinking hole 1333, and the second bearing 1222 in the third through-hole 1351 in sequence and then extends into the outflow channel 40. The first bearing 1221 and the second bearing 1222 are connected to the housing 13 by interference fit.

Optionally, the second bearing 1222 is located outside the first cavity 14. In the first direction x, the outflow channel 40 is located at one side of the second bearing 1222, and the first sinking hole 1333 is located at another side of the second bearing 1222. An outer diameter of the second bearing 1222 is greater than the diameter of the first sinking hole 1333. The second bearing 1222 is preferably a high-precision sliding bearing made of ceramic material (not limited to zirconia), and is designed with a clearance fit to the rotor shaft 121. The gap is controlled between 2 µm and 5 µm, and surface finish of an inner hole of the second bearing 1222and surface finish of an end face of the second bearing 1222 are as high as possible (controlled below Ra 0.4). When the motor body 10 is operating, the rotor shaft 121 rotates at a high speed. In this case, a fluid-floating hydrodynamic friction through the perfusion solution is formed between the rotor shaft 121 and the second bearing 1222 through the perfusion solution, and the second bearing 1222 has good temperature stability and wear resistance, such that the generated particles may be as few as possible.

In the motor assembly of the active interventional medical device provided by the embodiments, the first sinking hole 1333 communicates the first through-hole 1331 with the communication groove 1332, and the diameter of the first sinking hole 1333 is greater than the diameter of the first through-hole 1331, such that the perfusion solution converges at the first sinking hole 1333 to increase a pressure at the first sinking hole 1333, which reduces the probability of blood flowing into the motor body 10. When the perfusion of the perfusion catheter is interrupted, the perfusion solution in the first sinking hole 1333 can still maintain the pressure for a period to avoid blood entering the motor body 10 caused by the interruption of perfusion of the perfusion catheter. The second bearing 1222 is located outside the first cavity 14, and the second bearing 1222 is the sliding bearing. A gap of the sliding bearing is small, such that less perfusion solution flows into the outflow channel 40 through the second bearing 1222, and the total amount of blood in the outflow channel 40 flowing into the motor body 10 can also be reduced. The impeller 42 and the rotor shaft 121 form a cantilever beam structure, and a rotation stability of the impeller 42 is improved by disposing the second bearing 1222 closer to the impeller 42.

In some optional embodiments, the base plate 1341 is provided with a first wire pass-through hole 1344 penetrating the base plate 1341 along the first direction x, and the first wire pass-through hole 1344 is in communication with the second cavity 15.

Optionally, the cover body 135 is provided with a second wire pass-through hole 1352 penetrating the cover body 135 along the first direction x, the second wire pass-through hole 1352 communicates the second cavity 15 with the outflow channel 40, the first wire pass-through hole 1344 communicates the second cavity 15 with the interventional catheter, and the first wire pass-through hole 1344 is aligned with the second wire pass-through hole 1352. The wiring harness of the pressure sensor extends through the first wire pass-through hole 1344, the second wire pass-through hole 1352, and the second cavity 15, and is ultimately connected to the exterior of the patient through the interventional catheter.

In the motor assembly of the active interventional medical device provided by the embodiments, the base plate 1341 is provided with the first wire pass-through hole 1344 and the cover body 135 is provided with the second wire pass-through hole 1352, such that the wiring harness of the pressure sensor can pass through the second cavity 15, which significantly reduces the outer diameter of the motor assembly of the active interventional medical device compared with the solution where the wiring harness is disposed on the outer edge of the housing 13. In this way, the difficulty for inserting the motor assembly of the active interventional medical device into the patient's body is reduced.

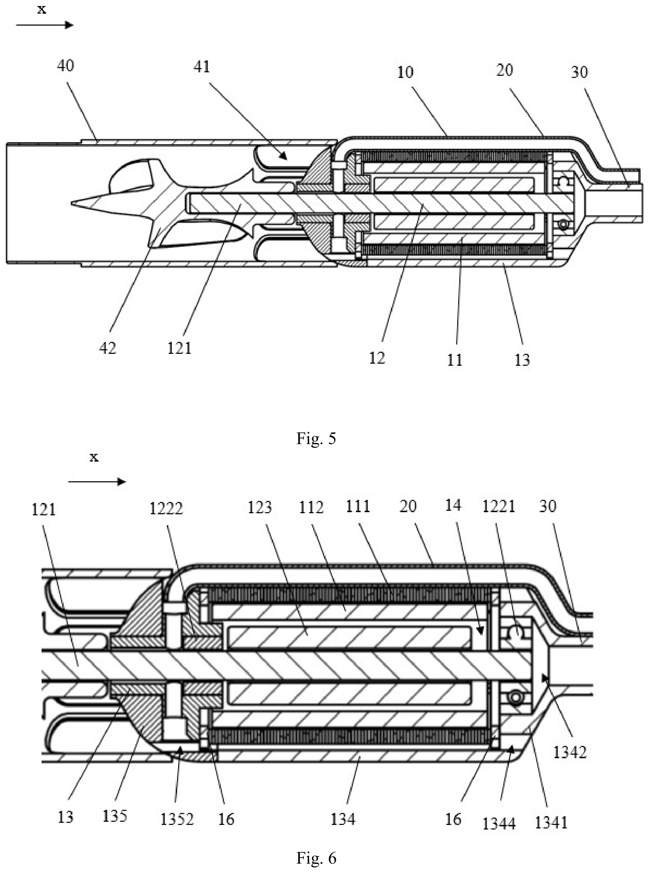

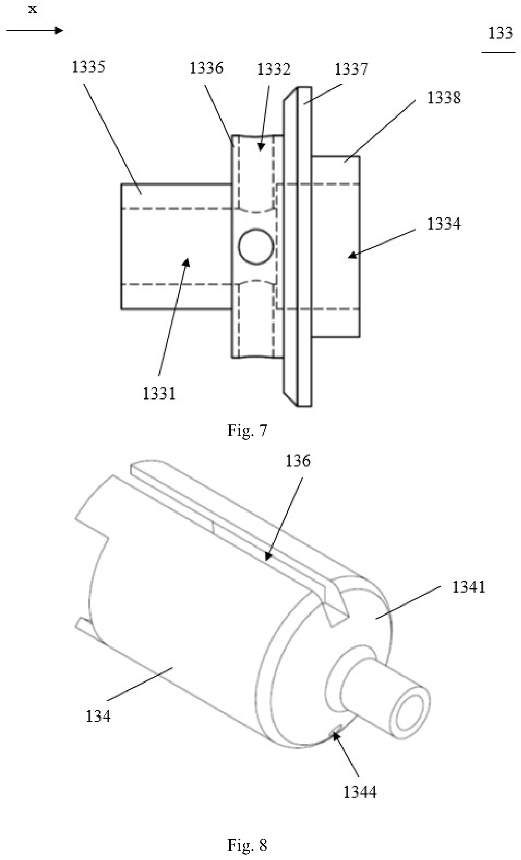

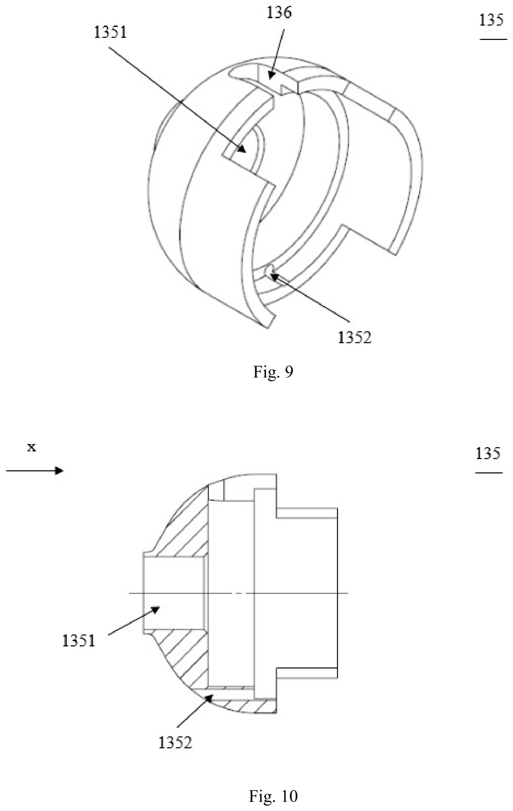

Referring to Figs. 5 to 10, Fig. 5 is a second cross-sectional view of a motor assembly of an active interventional medical device provided by some examples, Fig. 6 is a partially enlarged cross-sectional view of the motor assembly of the active interventional medical device in Fig. 5 provided by some examples, Fig. 7 is a schematic diagram of a sealing cover in Fig. 6 provided by some examples, Fig. 8 is a schematic diagram of a sleeve, a base plate, and a flow groove in Fig. 6 provided by some examples, Fig. 9 is a schematic diagram of a cover body and the flow groove in Fig. 6 provided by some examples, and Fig. 10 is a cross-sectional view of the cover body in Fig. 6 provided by some examples.

As shown in Figs. 5 to 10, in some optional embodiments, the second bearing 1222 is embedded in the sealing cover 133, and the perfusion solution flows through the perfusion catheter 20, the communication groove 1332, the second bearing 1222, the first bearing 1221, and the reflux catheter 30 in sequence in such a manner that the perfusion solution flows out of the first cavity 14.

Optionally, the sealing cover 133 is provided with a second sinking hole 1334 along the first direction x, and the second sinking hole 1334 is coaxial with the first through-hole 1331. A diameter of the second sinking hole 1334 is greater than the diameter of the first through-hole 1331. The second sinking hole 1334 is located at a side of the first through-hole 1331 away from the impeller 42. The communication groove 1332 communicates the perfusion catheter 20 with the first through-hole 1331. The second bearing 1222 is mounted to the second sinking hole 1334 by interference fit, that is, the second bearing 1222 is located in the first cavity 14.

Optionally, the sealing cover 133 is a stepped end cover, and includes a first step 1335, a second step 1336, a third step 1337, and a fourth step 1338 that are disposed in sequence in the first direction x. Diameters of the first step 1335, the second step 1336, and the third step 1337 increase in sequence, and a diameter of the fourth step 1338 is smaller than a diameter of the third step 1337. The sealing cover 133 is connected to the cover body 135, and a connection method includes a process such as welding and adhesive bonding. The first step 1335, the second step 1336, and the third step 1337 are fitted to corresponding holes in the cover body 135, and the fourth step 1338 extends into the first cavity 14. The perfusion solution flows into the sealing cover 133 through the communication groove 1332 and diverges at the first through-hole 1331. Most of the perfusion solution flows to the second sinking hole 1334 and flows into the first cavity 14 through the gap in the second bearing 1222, and finally flows out of the first cavity 14 through the first bearing 1221. A small part of the perfusion solution remains in the gap between the first through-hole 1331 and the rotor shaft 121, and is used to balance a difference between the pressure at the outflow channel 40 and the pressure at the first through-hole 1331 to reduce the amount of blood in the outflow channel 40 flowing into the motor body 10.

In the motor assembly of the active interventional medical device provided by the embodiments, the second bearing 1222 is also disposed in the first cavity 14, such that the perfusion solution can flush both the first bearing 1221 and the second bearing 1222. In this way, the total amount of particles flowing into the patient's body is further reduced, and the product safety is improved.

In some optional embodiments, an inner wall of the housing 13 is provided with a flow groove 136 recessed towards the rotor shaft 121, the flow groove 136 is in communication with the first cavity 14, and at least part of the flow groove 136 serves as a channel for the perfusion catheter 20.

Optionally, the sleeve 134 and the cover body 135 each are provided with the flow groove 136 recessed towards the rotor shaft 121, and extending along the first direction x, and the flow groove 136 is in directly communication with the communication groove 1332, that is, the perfusion solution does not flow through the second cavity 15.

In the motor assembly of the active interventional medical device provided by the embodiments, the perfusion catheter 20 is arranged in the flow groove 136 inwardly recessed and provided on the housing 13 to reduce the outer diameter of the housing 13, thereby reducing the difficulty for inserting the motor assembly of the active interventional medical device into the patient's body.

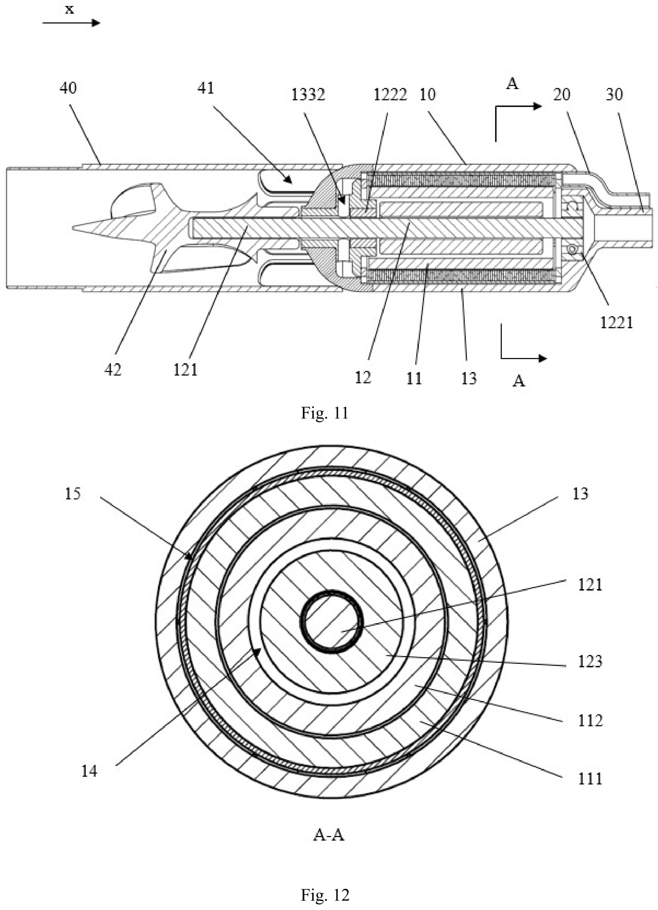

Referring to Figs. 11 and 12, Fig. 11 is a third cross-sectional view of a motor assembly of an active interventional medical device provided by some examples, and Fig. 12 is a cross-sectional view of the motor assembly of the active interventional medical device along A-A in Fig. 11 provided by some examples.

As shown in Figs. 11 and 12, in some optional embodiments, the sealing cover 133 is the above stepped end cover, and the second bearing 1222 is located in the first cavity 14. Both the perfusion catheter 20 and the communication groove 1332 are in communication with the second cavity 15, and the perfusion solution flows into the first cavity 14 through the second cavity 15 and the communication groove 1332, flushes the first bearing 1221 and the second bearing 1222 in such a manner that the perfusion solution flows out of the first cavity 14.

Optionally, a distance between the stator assembly 11 and the rotor assembly 12 and a distance between the stator assembly 11 and the housing 13 can be set according to the actual situation, that is, a volume of the first cavity 14 and a volume of the second cavity 15 can be set according to the actual situation. For example, in order to reduce the outer diameter of the housing 13, the distance between the housing 13 and the stator assembly 11 is reduced, and thus the volume of the second cavity 15 is reduced.

In view of the above, the motor assembly of the active interventional medical device provided by the embodiments includes the motor body 10, the perfusion catheter, and the reflux catheter 30. The motor body 10 includes the stator assembly 11, the rotor assembly 12, and the housing 13, and the stator assembly 11 and the rotor assembly 12 are disposed in the housing 13. The gap between the rotor assembly 12 and the stator assembly 11 forms the first cavity 14. The rotor assembly 12 includes the rotor shaft 121 and the bearing 122. The rotor shaft 121 is molded by extending in the first direction x. The bearing 122 is fitted on the rotor shaft 121. At least part of the perfusion catheter 20 is disposed in the housing 13, and the perfusion catheter 20 is in communication with the first cavity 14. At least part of the reflux catheter 30 is disposed in the housing 13, and the reflux catheter 30 is in communication with the first cavity 14. The reflux catheter 30 is isolated from the perfusion catheter 20. The external perfusion solution flows through the perfusion catheter 20, the bearing 122, and the reflux catheter 30 in sequence in such a manner that the external perfusion solution flows out of the first cavity 14. In the present disclosure, the perfusion solution is perfused into the first cavity 14 through the perfusion catheter 20, which reduces the particles generated when the motor body 10 runs. The perfusion solution in the first cavity 14 is recycled by the reflux catheter 30, and at the same time, the bearing 122 is flushed with the perfusion solution, which carries the particles generated in the motor body 10 out of the human body, reduces the harm to the human body, and improves the product safety. In addition, the perfusion solution is recycled after flowing through the interior of the motor body 10, which can bring out the heat generated by the blood-pumping motor and reduce the temperature rise of the motor body 10.

The above merely illustrates specific implementations of the present disclosure, those skilled in the art can clearly understand that, for the convenience and brevity of the description, the specific working processes of the above systems, modules, and units can be referred to the corresponding processes in the foregoing method embodiments, which will not repeated herein. It should be understood that, the protection scope of the present disclosure is not limited to the above contents, and various equivalent modifications or replacements can be easily though by those skilled in the art within the technical scope disclosed by the present disclosure, and these modifications or replacements should all fall within the protection scope of the present disclosure.

## Claims

1. A motor assembly of an active interventional medical device, comprising:
a motor body, comprising:
a rotor assembly comprising a rotor shaft extending in a first direction and a bearing fitted on the rotor shaft,
a stator assembly fitted on the rotor assembly, a gap between the rotor assembly and the stator assembly forming a first cavity, and
a housing in which the stator assembly and the rotor assembly are disposed, the rotor shaft being rotatable relative to the housing through the bearing;
a perfusion catheter in communication with the first cavity, at least part of the perfusion catheter being disposed in the housing; and
a reflux catheter isolated from the perfusion catheter and in communication with the first cavity, at least part of the reflux catheter being disposed in the housing, so that external perfusion solution flows through the perfusion catheter, the bearing, and the reflux catheter in sequence in such a manner that the external perfusion solution flows out of the first cavity.

2. The motor assembly of the active interventional medical device according to claim 1, wherein an inner wall of the housing is provided with a flow groove recessed towards the rotor shaft, the flow groove being in communication with the first cavity, and at least part of the flow groove serving as a channel for the perfusion catheter.

3. The motor assembly of the active interventional medical device according to claim 1, wherein the perfusion catheter is disposed at a proximal end of the housing, a gap between the stator assembly and the housing forms a second cavity, and the first cavity is in communication with the perfusion catheter through the second cavity.

4. The motor assembly of the active interventional medical device according to claim 3, wherein the gap between the stator assembly and the housing is divided into two compartments of which one serves as the second cavity and the other one serves as a cavity for accommodating a wiring harness.

5. The motor assembly of the active interventional medical device according to claim 1, wherein the rotor shaft extends out of the housing from a distal end of the housing; and
wherein the housing comprises a sealing cover located at the distal end of the housing, fitted on the rotor shaft, and sealing the distal end of the housing.

6. The motor assembly of the active interventional medical device according to claim 5, wherein a side of the sealing cover facing towards an interior of the housing is provided with a communication groove communicating the first cavity with the perfusion catheter.

7. The motor assembly of the active interventional medical device according to claim 6, wherein the rotor assembly comprises a plurality of the bearings which comprises:
a first bearing fitted on the rotor shaft and disposed at a proximal end of the housing, and
a second bearing fitted on the rotor shaft and disposed at the distal end of the housing,
so that the perfusion solution flows through the perfusion catheter, the communication groove, the first bearing, and the reflux catheter in sequence in such a manner that the perfusion solution flows out of the first cavity.

8. The motor assembly of the active interventional medical device according to claim 7, wherein the second bearing is embedded in the sealing cover, so that the perfusion solution flows through the perfusion catheter, the communication groove, the second bearing, the first bearing, and the reflux catheter in sequence in such a manner that the perfusion solution flows out of the first cavity.

9. The motor assembly of the active interventional medical device according to claim 7, wherein the second bearing is located at a side of the sealing cover away from the first cavity.

10. The motor assembly of the active interventional medical device according to claim 1, wherein the housing further comprises:
a base plate located at a proximal end of the housing, isolating the stator assembly and the rotor assembly from external environment, and provided with a second through-hole penetrating the base plate along the first direction,
wherein the first cavity is in communication with the reflux catheter through the second through-hole.

11. The motor assembly of the active interventional medical device according to claim 10, wherein the reflux catheter and the rotor shaft are coaxial, and the reflux catheter is disposed on an extension line of the rotor shaft extending towards the proximal end of the housing.
